(19) Europäisches Patentamt
European Patent Office
Office européen des brevets

(11) EP 3 653 104 B1

(12) **EUROPEAN PATENT SPECIFICATION**

(45) Date of publication and mention of the grant of the patent:
**25.08.2021 Bulletin 2021/34**

(21) Application number: **18831899.2**

(22) Date of filing: **11.07.2018**

(51) Int Cl.:
***A61B 1/005*** *(2006.01)* ***G02B 23/24*** *(2006.01)*

(86) International application number:
**PCT/JP2018/026153**

(87) International publication number:
**WO 2019/013243 (17.01.2019 Gazette 2019/03)**

(54) **FLEXIBLE TUBE FOR ENDOSCOPE, ENDOSCOPIC MEDICAL DEVICE, AND METHODS FOR PRODUCING THESE**

SCHLAUCH FÜR EIN ENDOSKOP, ENDOSKOPARTIGES MEDIZINPRODUKT UND HERSTELLUNGSVERFAHREN FÜR DIESE

TUBE FLEXIBLE POUR ENDOSCOPE, DISPOSITIF MÉDICAL DE TYPE ENDOSCOPE ET PROCÉDÉS DE PRODUCTION DE CEUX-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.07.2017 JP 2017136025**

(43) Date of publication of application:
**20.05.2020 Bulletin 2020/21**

(73) Proprietor: **FUJIFILM Corporation**
**Tokyo 106-8620 (JP)**

(72) Inventors:
- **YOSHITANI, Toshihide**
  **Ashigarakami-gun**
  **Kanagawa 258-8577 (JP)**
- **ABE, Shinya**
  **Ashigarakami-gun**
  **Kanagawa 258-8577 (JP)**
- **NAKAI, Yoshihiro**
  **Ashigarakami-gun**
  **Kanagawa 258-8577 (JP)**

(74) Representative: **Hoffmann Eitle**
**Patent- und Rechtsanwälte PartmbB**
**Arabellastraße 30**
**81925 München (DE)**

(56) References cited:
**EP-A1- 3 000 834**
**JP-A- H10 317 159**
**JP-A- 2003 534 440**
**JP-A- 2004 141 487**
**JP-A- 2011 056 710**
**JP-A- 2015 214 743**
**JP-A- 2016 067 566**
**US-B1- 6 350 799**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### BACKGROUND OF THE INVENTION

1. Field of the Invention

The present invention relates to a flexible tube for an endoscope, an endoscopic medical device, and methods for producing the same.

2. Description of the Related Art

Endoscopes are medical devices for observing the inside of body cavities of patients. Since endoscopes are used by being inserted into the body cavities, there is a requirement for endoscopes that do not damage organs or does not cause pain and discomfort to patients. In view of such a requirement, a spiral tube formed by winding a soft, flexible metal strip in a spiral form is used as a flexible tube that forms an insertion section (structural section inserted into a body cavity) of an endoscope. Furthermore, the periphery of the spiral tube is covered with a soft resin, and this resin cover layer is covered with a topcoat layer, as needed, so as not to provide stimulation or damage to the inner surface of the body cavity such as the esophagus or the intestines.

The resin cover layer can be formed by extrusion-molding a resin on an outer peripheral surface of a flexible tube base that is formed by covering a spiral tube with a cylindrical mesh. In this case, it is preferable to make the distal end side soft so as to be easily inserted into the body cavity and to make the proximal end side hard so as to enhance operability. In consideration of this point, it has been proposed that a two-layer structure having an inner layer and an outer layer that have different degrees of hardness is used as the resin cover layer, and a ratio of the thickness of the inner layer to the thickness of the outer layer is changed in the axial direction of the flexible tube.

To improve operability, durability, and the like of an endoscope, it is important to enhance adhesiveness between a flexible tube base and a resin cover layer that covers the flexible tube base. If this adhesiveness is insufficient, when a flexible tube is inserted into a body cavity, a wrinkle is easily generated on the resin cover layer due to bending of the flexible tube. In addition, when the flexible tube is rotated in the body cavity, twisting of the resin cover layer easily occurs. When such a wrinkle or twisting is generated in the resin cover layer, for example, the surface of the flexible tube catches the inside of the body cavity, which may cause a pain to a subject.

As a technique for addressing this problem, JP1999-42205A (JP-H11-42205A) discloses that a silane coupling agent that exhibits adhesiveness to both inorganic substances and organic substances is disposed between a flexible tube base and a resin cover layer.

Furthermore, high resilience is required for a flexible tube in order to smoothly move the flexible tube in the body cavity. By enhancing resilience of the flexible tube, the flexible tube that has passed through a bent portion in the body cavity is easily returned to a linear state, and the load on a subject during a test can be further reduced.

EP 3 000 834 A1 relates to a flexible tube for an endoscope, adhesive for an endoscope, endoscope-type medical device, as well as method of producing a flexible tube for an endoscope and method of producing an endoscope-type medical device. The flexible tube for an endoscope contains: a tubular flexible tube substrate material having a flexibility; and a resin layer covering the flexible tube substrate material, in which the resin layer is adhered to the flexible tube substrate material with an adhesive hardened, the adhesive hardened contains an ester-based polyurethane resin, which is a hardened resin of an adhesive for an endoscope, and the adhesive for an endoscope contains an ester-based urethane polymer having a structure represented by a specific formula. US 6,350,799 B1 refers to a coolant resistant and thermally stable primer composition, comprising an organic-functional silane, preferably the reaction product of at least one amino-functional silane and at least one isocyanato-functional silane, is provided. The primer composition may additionally comprise a phenoxy resin, a phenolic resin and talc. A method for bonding an elastomer to a metal also is provided.

JP 2004 141 487 A refers to a flexible tube for an endoscope. In the flexible tube for the endoscope which is constructed by applying an outer coating to the surface of a flexible tube material, the outer coating is composed of: a resin which is obtained by applying hydrolysis preventing treatment to a hard segment of a polyester elastomer; or a polyester elastomer using polybutylene naphthalate for the hard segment.

### SUMMARY OF THE INVENTION

An object of the present invention is to provide a flexible tube for an endoscope, the flexible tube having good adhesiveness between a flexible tube base and a resin cover layer that covers the flexible tube base and good resilience, and an endoscopic medical device that includes the flexible tube for an endoscope. Another object of the present invention is to provide a method for producing the flexible tube for an endoscope and a method for producing the endoscopic

medical device.

The inventors of the present invention have conducted extensive studies on formation of a resin cover layer in a flexible tube for an endoscope. As a result, it has been found that when a primer layer that includes a silane coupling agent with a specific structure having an amino group is formed on a surface of a flexible tube base formed of a metal material, and a polyurethane elastomer is used as a constituent material of a resin cover layer that is in contact with the primer layer, adhesiveness between the flexible tube base and the resin cover layer in the resulting flexible tube can be enhanced to a desired sufficient level, and the flexible tube also has good resilience. The inventors have further conducted studies on the basis of these findings and completed the present invention.

The objects of the present invention have been achieved by the following means.

[1] A flexible tube for an endoscope, the flexible tube having a flexible tube base containing metal as a constituent material; a resin cover layer that covers an outer periphery of the flexible tube base; and a primer layer that includes a compound represented by general formula (1) below and that is disposed between the flexible tube base and the resin cover layer, in which the resin cover layer includes a polyurethane elastomer at least on a side in contact with the primer layer.

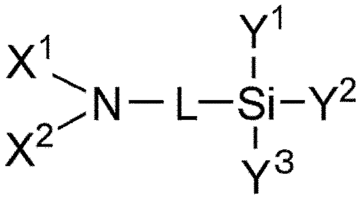

General formula (1)

In the formula, $X^1$ and $X^2$ each represent a hydrogen atom, an alkyl group, a cycloalkyl group, an alkenyl group, an aryl group, an acyl group, an alkoxycarbonyl group, or a carbamoyl group.

$Y^1$ represents a hydroxy group or an alkoxy group. $Y^2$ and $Y^3$ each represent a hydroxy group, an alkoxy group, or an alkyl group.

L represents a single bond, a divalent group selected from the group consisting of an alkylene group, an arylene group, and -O-, or a divalent group which is a combination of two or more divalent groups selected from the group consisting of an alkylene group, an arylene group, and -O-.

[2] The flexible tube for an endoscope according to [1], in which the metal that constitutes the flexible tube base is stainless steel.

[3] The flexible tube for an endoscope according to [1] or [2], in which the metal that constitutes the flexible tube base has a passivation film on a surface of the flexible tube base.

[4] The flexible tube for an endoscope according to any one of [1] to [3], in which the resin cover layer has a single-layer structure or a multilayer structure and includes the polyurethane elastomer in a layer in contact with the primer layer.

[5] The flexible tube for an endoscope according to any one of [1] to [4], in which the resin cover layer has a two-layer structure, and a ratio of a thickness of an inner layer to a thickness of an outer layer of the two-layer structure changes in a gradient manner in an axial direction of the flexible tube base.

[6] The flexible tube for an endoscope according to [5], in which the ratio of the thickness of the inner layer to the thickness of the outer layer is inner layer:outer layer = 5:95 to 40:60 on one end of the flexible tube for an endoscope and inner layer:outer layer = 95:5 to 60:40 on the other end.

[7] An endoscopic medical device having the flexible tube for an endoscope according to anyone of [1] to [6].

[8] A method for producing a flexible tube for an endoscope, the method including a step of forming, on at least an outer periphery of a flexible tube base that contains metal as a constituent material, a primer layer that includes a compound represented by general formula (1) below; and a step of forming a resin cover layer by covering, with a resin that includes a polyurethane elastomer, the primer layer formed on the outer periphery of the flexible tube base so as to be in contact with the primer layer.

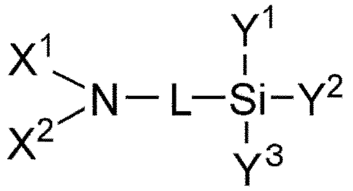

General formula (1)

In the formula, $X^1$ and $X^2$ each represent a hydrogen atom, an alkyl group, a cycloalkyl group, an alkenyl group, an aryl group, an acyl group, an alkoxycarbonyl group, or a carbamoyl group.

$Y^1$ represents a hydroxy group or an alkoxy group. $Y^2$ and $Y^3$ each represent a hydroxy group, an alkoxy group, or an alkyl group.

L represents a single bond, a divalent group selected from the group consisting of an alkylene group, an arylene group, and -O-, or a divalent group which is a combination of two or more divalent groups selected from the group consisting of an alkylene group, an arylene group, and -O-.

[9] The method for producing a flexible tube for an endoscope according to [8], in which the resin cover layer has a two-layer structure, at least an inner layer of the two-layer structure includes a polyurethane elastomer, and a ratio of a thickness of the inner layer to a thickness of an outer layer of the two-layer structure changes in a gradient manner in an axial direction of the flexible tube base.

[10] A method for producing an endoscopic medical device, including a step of producing a flexible tube for an endoscope by the method for producing a flexible tube for an endoscope according to [8] or [9]; and a step of incorporating the produced flexible tube for an endoscope into an insertion section of an endoscopic medical device.

[11] A method for producing an endoscopic medical device, including incorporating the flexible tube for an endoscope according to any one of [1] to [6] into an insertion section of an endoscopic medical device.

Herein, when a plurality of substituents, linking groups, or the like (hereinafter referred to as substituents or the like) represented by specific symbols are present or a plurality of substituents or the like are defined simultaneously or alternatively, the substituents or the like may be the same or different from each other. In addition, even if not specifically stated, when a plurality of substituents or the like are adjacent to each other, they may be linked or fused to each other to form a ring.

Herein, a substituent (the same applies to a linking group) in which substitution or no substitution is not specified may have any substituent within the range in which desired effects are achieved. The same applies to a compound in which substitution or no substitution is not specified.

Herein, when the number of carbon atoms of a group is specified, the number of carbon atoms means the number of carbon atoms of the whole group. That is, in the case of a form where the group further has a substituent, the number of carbon atoms means the number of carbon atoms of the whole that includes this substituent.

The flexible tube for an endoscope according to the present invention has good adhesiveness between a flexible tube base and a resin cover layer that covers the flexible tube base and good resilience.

According to the endoscopic medical device according to the present invention, a flexible tube which is a structural section inserted into a body cavity has good adhesiveness between a flexible tube base and a resin cover layer that covers the flexible tube base and good resilience. Therefore, the endoscopic medical device according to the present invention can further reduce the load on a subject during use.

The method for producing a flexible tube for an endoscope according to the present invention can provide a flexible tube for an endoscope, the flexible tube having good adhesiveness between a flexible tube base and a resin cover layer that covers the flexible tube base and good resilience.

According to the method for producing an endoscopic medical device according to the present invention, a flexible tube that forms the device can have good adhesiveness between a flexible tube base and a resin cover layer that covers the flexible tube base, and resilience of this flexible tube can also be enhanced. Therefore, the method for producing an endoscopic medical device according to the present invention can provide an endoscopic medical device in which the load on a subject during use is further reduced.

## BRIEF DESCRIPTION OF THE DRAWINGS

Fig. 1 is an external view illustrating a configuration of an electronic endoscope according to an embodiment;

Fig. 2 is a partial sectional view illustrating a configuration of a flexible tube for an endoscope according to an

embodiment;

Fig. 3 is a block diagram illustrating a configuration of an apparatus for producing a flexible tube for an endoscope according to an embodiment; and

Fig. 4 is a sectional view taken along line B-B in Fig. 3.

## DESCRIPTION OF THE PREFERRED EMBODIMENTS

An endoscopic medical device according a preferred embodiment of the present invention will be described using an electronic endoscope as an example. An electronic endoscope includes a flexible tube for an endoscope (hereinafter, a flexible tube for an endoscope may be simply referred to as a "flexible tube"), the flexible tube being incorporated in the electronic endoscope, and is used as a medical device for, for example, observing the inside of a body cavity by inserting the flexible tube into the body cavity. In an example illustrated in Fig. 1, an electronic endoscope 2 includes an insertion section 3 that is inserted into a body cavity, a main body operating section 5 that is connected to a proximal end portion of the insertion section 3, and a universal cord 6 that is connected to a processor device and a light source device. The insertion section 3 includes a flexible tube 3a that is connected to the main body operating section 5, an angle portion 3b connected to the flexible tube 3a, and a tip portion 3c which is connected to a tip of the angle portion 3b and in which an imaging device (not shown) for imaging the inside of the body cavity is installed. The flexible tube 3a that accounts for a large portion of the length of the insertion section 3 has flexibility across substantially the entire length thereof and is configured so that, in particular, a portion that is inserted into the inside of a body cavity or the like has higher flexibility.

### Flexible Tube Base

The flexible tube has, as an innermost layer, a flexible tube base containing metal as a constituent material.

As illustrated in Fig. 2, a flexible tube base 14 preferably has a form in which a spiral tube 11 formed, on the innermost side, by winding a metal strip 11a in a spiral form is covered with a cylindrical mesh 12 formed by braiding metal wires, and caps 13 are fitted in both ends of the resulting product. The metal constituting the flexible tube base 14 preferably has a surface that has been subjected to a passivation treatment in order to prevent corrosion. That is, the flexible tube base 14 preferably has a passivation film on an outer periphery thereof. This passivation treatment can be performed by an ordinary method. A passivation film can be formed on a surface of metal by, for example, immersing the metal in a solution including a strong oxidizing agent such as nitric acid, heating the metal in air (oxygen) or water (water vapor), or anodizing the metal in a solution including an oxidizing agent.

The metal constituting the flexible tube base 14 is preferably stainless steel. A surface of stainless steel is usually in a state in which a passivation film is formed by bonding between chromium and oxygen. However, even in the case where stainless steel is used as a constituent material of the flexible tube base 14, the stainless steel is preferably subjected to the passivation treatment described above so that a more uniform passivation film is more reliably formed over the entire surface of the stainless steel.

### Primer Layer

In the present invention, a primer layer (not shown) is disposed on an outer periphery of the flexible tube base. The presence of this primer layer can effectively enhance adhesiveness between the flexible tube base and a resin cover layer which will be described below, the resin cover layer being disposed so as to cover the outer periphery of the flexible tube base. In the present invention, this primer layer includes a compound represented by general formula (1) below.

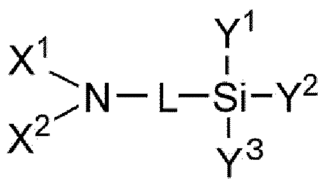

General formula (1)

In general formula (1), $X^1$ and $X^2$ each represent a hydrogen atom, an alkyl group, a cycloalkyl group, an alkenyl group, an aryl group, an acyl group, an alkoxycarbonyl group, or a carbamoyl group.

The alkyl groups for $X^1$ and $X^2$ may be linear or branched. The number of carbon atoms of each of the alkyl groups is preferably an integer of 1 to 20, more preferably 1 to 15, still more preferably 1 to 10, and particularly preferably

1 to 8.

Specific examples of the alkyl groups for $X^1$ and $X^2$ include methyl, ethyl, n-propyl, isopropyl, n-butyl, s-butyl, isobutyl, t-butyl, n-pentyl, n-hexyl, n-heptyl, and n-octyl.

The number of carbon atoms of each of the cycloalkyl groups for $X^1$ and $X^2$ is preferably 3 to 20, more preferably 3 to 15, still more preferably 3 to 10, and particularly preferably 3 to 8. Specific examples of the cycloalkyl groups for $X^1$ and $X^2$ include cyclopentyl and cyclohexyl.

The alkenyl groups for $X^1$ and $X^2$ may be linear or branched. The number of carbon atoms of each of the alkenyl groups is preferably an integer of 2 to 20, more preferably 2 to 15, still more preferably 2 to 10, and particularly preferably 2 to 5.

The number of carbon atoms of each of the aryl groups for $X^1$ and $X^2$ is preferably an integer of 6 to 20, more preferably 6 to 15, still more preferably 6 to 12, and particularly preferably 6 to 10.

Specific examples of the aryl groups for $X^1$ and $X^2$ include phenyl and naphthyl. The aryl group is preferably phenyl.

The number of carbon atoms of each of the acyl groups for $X^1$ and $X^2$ is preferably an integer of 2 to 40, more preferably 2 to 30, still more preferably 2 to 20, and particularly preferably 2 to 15. In the present invention, the acyl groups include alkylcarbonyl groups and arylcarbonyl groups.

The number of carbon atoms of each of the alkoxycarbonyl groups for $X^1$ and $X^2$ is preferably an integer of 2 to 40, more preferably 2 to 30, still more preferably 2 to 20, and particularly preferably 2 to 15.

The number of carbon atoms of each of the carbamoyl groups for $X^1$ and $X^2$ is preferably an integer of 1 to 40, more preferably 1 to 30, still more preferably 1 to 20, and particularly preferably 1 to 15.

When the alkyl groups, cycloalkyl groups, alkenyl groups, aryl groups, acyl groups, alkoxycarbonyl groups, and carbamoyl groups for $X^1$ and $X^2$ have substituents, the substituents preferably include an amino group (preferably an amino group having a hydrogen atom bonded to a nitrogen atom, and more preferably an unsubstituted amino group), a hydroxy group, and/or a silyl group (preferably a silyl group having a substituent, and the number of carbon atoms of the whole of substituents (when the silyl group has three substituents, the whole of the three substituents) in the substituted silyl group is preferably 1 to 10 and more preferably 1 to 6. A silyl group having at least one alkoxy group as a substituent is preferred) (that is, at least one of the amino group, the hydroxy group, or the silyl group is preferably included in the substituents).

When the alkyl groups, cycloalkyl groups, alkenyl groups, aryl groups, acyl groups, alkoxycarbonyl groups, and carbamoyl groups for $X^1$ and $X^2$ have a substituent, it is also preferred that the substituent be an amino group (preferably an amino group having a hydrogen atom bonded to a nitrogen atom, and more preferably an unsubstituted amino group), a hydroxy group, or a silyl group (preferably a silyl group having a substituent, and the number of carbon atoms of the whole of substituents (when the silyl group has three substituents, the whole of the three substituents) in the substituted silyl group is preferably 1 to 10, and more preferably 1 to 6. A silyl group having at least one alkoxy group as a substituent is preferred).

$X^1$ and $X^2$ may be linked to each other to form a ring. The number of atoms constituting this ring is preferably 3 to 10, more preferably 4 to 8, and preferably 5 or 6. The ring that can be formed by linking $X^1$ and $X^2$ to each other usually includes, as ring-constituting atoms, a carbon atom besides a nitrogen atom. Alternatively, the ring may have a heteroatom other than a nitrogen atom. Examples of the heteroatom other than a nitrogen atom include an oxygen atom, a sulfur atom, and a silicon atom.

$X^1$ and $X^2$ are each preferably a hydrogen atom. It is also preferred that one of $X^1$ and $X^2$ be a hydrogen atom and the other of $X^1$ and $X^2$ be an alkyl group having an amino group.

$Y^1$ represents a hydroxy group or an alkoxy group and is preferably an alkoxy group.

Examples of an alkyl group that forms the alkoxy group for $Y^1$ include the alkyl groups for $X^1$ and $X^2$. Preferred forms of the alkyl group are also the same as those of the alkyl groups for $X^1$ and $X^2$.

$Y^2$ and $Y^3$ each represent a hydroxy group, an alkoxy group, or an alkyl group. The alkoxy groups for $Y^2$ and $Y^3$ each have the same definition as the alkoxy group for $Y^1$, and preferred forms of each of the alkoxy groups are also the same as those of the alkoxy group for $Y^1$. The alkyl groups for $Y^2$ and $Y^3$ have the same definition as the alkyl groups for $X^1$ and $X^2$, and preferred forms of the alkyl groups are also the same as those of the alkyl groups for $X^1$ and $X^2$.

Among $Y^1$, $Y^2$, and $Y^3$, at least one is preferably an alkoxy group, at least two are more preferably alkoxy groups, and all of $Y^1$, $Y^2$, and $Y^3$ are still more preferably alkoxy groups.

In general formula (1), $X^2$ and $Y^3$ may be linked to each other to form a ring. The number of atoms constituting this ring is preferably 3 to 10, more preferably 4 to 8, and preferably 5 or 6. When $X^2$ and $Y^3$ are linked to each other to form a ring, L described below is preferably a single bond. In the ring that can be formed by linking $X^2$ and $Y^3$ to each other, a ring-constituting atom other than a nitrogen atom and a silicon atom is preferably a carbon atom.

L is a single bond, a divalent group selected from the group consisting of an alkylene group, an arylene group, and -O- (an ether bond), or a divalent group which is a combination of divalent groups selected from the group consisting of an alkylene group, an arylene group, and -O-. When L is a divalent group, the molecular weight of L is preferably 14

to 300 and more preferably 14 to 210.

The alkylene group for L may be linear or branched. The number of carbon atoms of this alkylene group is preferably an integer of 1 to 20, more preferably 1 to 15, still more preferably 1 to 12, and particularly preferably 1 to 8.

The number of carbon atoms of the arylene group for L is preferably an integer of 6 to 20, more preferably 6 to 15, still more preferably 6 to 12, and particularly preferably 6 to 10. The arylene group for L is particularly preferably phenylene.

L is preferably an alkylene group and more preferably a linear alkylene group. The number of carbon atoms of this linear alkylene group is preferably an integer of 1 to 12 and more preferably 1 to 6.

The compound represented by general formula (1) and included in the primer layer functions as a silane coupling agent. Specifically, it is considered that "$-Si(Y^1)(Y^2)Y^3$" in general formula (1) interacts with metal that forms the flexible tube base, and "$-N(X^1)X^2$" interacts with a polyurethane elastomer that forms the resin cover layer, and consequently, the resin cover layer that coves the outer periphery of the flexible tube base can be strongly brought into close contact with the surface of the flexible tube base. An example of the interaction of "$-Si(Y^1)(Y^2)Y^3$" to the metal that forms the flexible tube base 14 is a condensation polymerization reaction between a hydroxy group generated by hydrolysis of an alkoxy group bonded to the Si atom of this group or a hydroxy group bonded to the Si atom and, for example, a hydroxy group on the surface of the metal of the flexible tube base.

Although the interaction between "$-N(X^1)X^2$" and a polyurethane elastomer is not necessarily clear, it is considered that a hydrogen bond between $-N(X^1)X^2$ and a urethane bond, a covalent bond between $-N(X^1)X^2$ and an isocyanate residue in the polyurethane elastomer, and the like are efficiently generated and contribute to the improvement in adhesiveness to the resin cover layer.

Heretofore, in order to improve adhesiveness between a flexible tube base and a resin cover layer, formation of an adhesive layer between the flexible tube base and the resin cover layer has been widely performed. A known example of this adhesive layer is an adhesive layer formed of a composition that contains a polymer such as a polyurethane and a polyisocyanate compound. This adhesive layer is a soft layer having a certain thickness.

In contrast, the compound represented by general formula (1) and used in the present invention contributes to adhesion between the flexible tube base and the resin cover layer in the monomolecular form, and the thickness of the primer layer is significantly smaller than that of a typical adhesive layer (in other words, the concept of the thickness cannot be conceived). That is, the primer layer that includes the compound represented by general formula (1) differs from the adhesive layer that requires a certain thickness and softness for adhesion between the flexible tube base and the resin cover layer. Therefore, the primer layer does not substantially affect resilience of the flexible tube, and the flexible tube according to the present invention also has good resilience.

Specific examples of the compound represented by general formula (1) are shown below. However, the present invention is not limited to the specific examples. In the structures below, Me represents methyl, and Et represents ethyl.

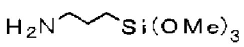
S-1

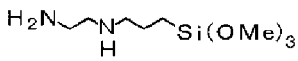
S-2

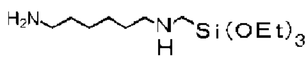
S-3

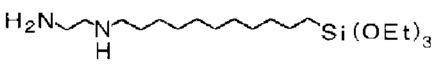
S-4

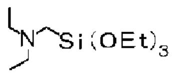
S-5

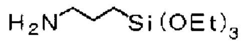
S-6

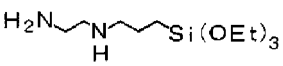
S-7

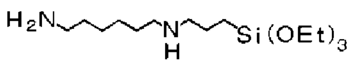
S-8

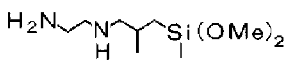
S-9

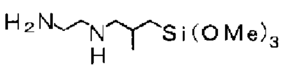
S-10

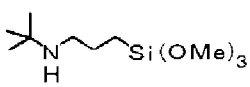
S-11

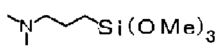
S-12

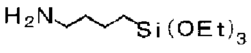

S-13

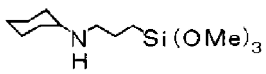

S-14

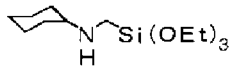

S-15

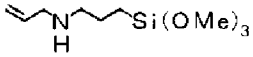

S-16

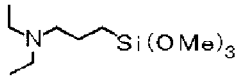

S-17

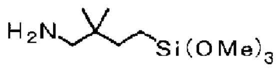

S-18

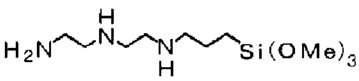

S-19

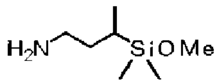

S-20

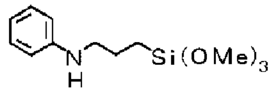

S-21

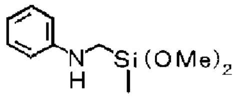

S-22

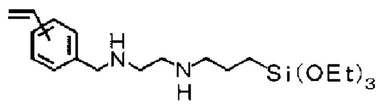

S-23

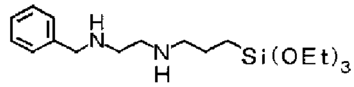

S-24

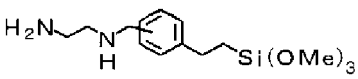

S-25

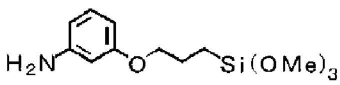

S-26

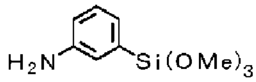

S-27

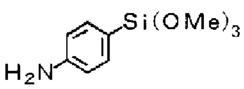

S-28

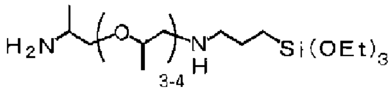

S-29

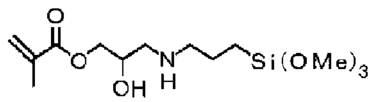

S-30

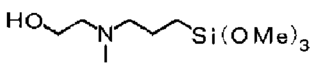

S-31

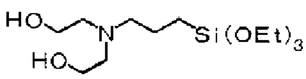

S-32

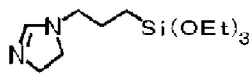

S-33

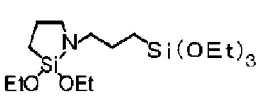

S-34

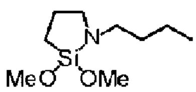

S-35

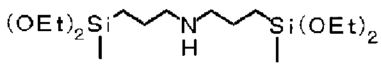

S-36

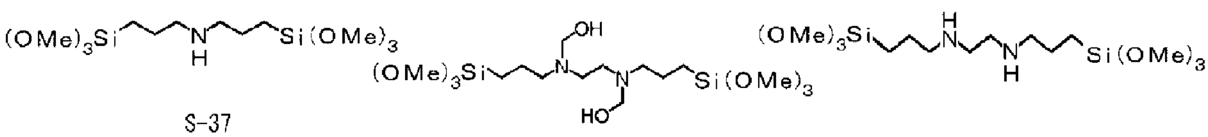

S-37

S-38

S-39

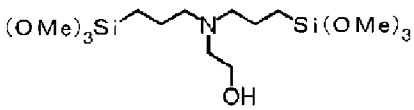

S-40

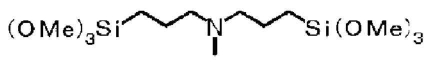

S-41

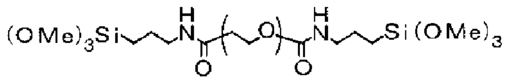

S-42

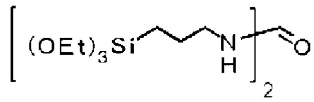

S-43

Resin Cover Layer

The flexible tube according to the present invention has a resin cover layer disposed on an outer periphery of a flexible tube base having a primer layer thereon.

In the embodiment in Fig. 2, an outer surface of a resin cover layer 15 is coated with a topcoat layer 16 that contains fluorine or the like and that contributes to, for example, chemical resistance. In Fig. 2, only a single spiral tube 11 is illustrated. Alternatively, the spiral tube 11 may be formed by concentrically stacking two or more layers. Note that the resin cover layer 15 and the topcoat layer 16 in the figure are shown to be thicker than the actual thicknesses with respect to the diameter of a flexible tube base 14 for the sake of clearly illustrating the layer structure.

In the present invention, the resin cover layer covers an outer peripheral surface of the flexible tube base having the above-described primer layer thereon. The resin cover layer 15 in the embodiment in Fig. 2 has a two-layer structure in which an inner layer 17 that covers the entire peripheral surface around the axis of the flexible tube base 14 and an outer layer 18 that covers the entire peripheral surface around the axis of the inner layer 17 are laminated. Typically, a soft resin is used as the material of the inner layer 17, and a hard resin is used as the material of the outer layer 18. However, the present invention is not limited to these embodiments.

In the present invention, when the resin cover layer has a multilayer structure having two or more layers, at least the innermost layer (layer that is in contact with the primer layer) includes a polyurethane elastomer, as described below. In the present invention, when the resin cover layer is a single layer, this single-layer resin cover layer includes a polyurethane elastomer. That is, the resin cover layer in the present invention includes a polyurethane elastomer at least on the side of the resin cover layer in contact with the primer layer.

Polyurethane Elastomer

Typical polyurethane elastomers that can be used for forming flexible tubes can be adopted as the polyurethane elastomer used in the resin cover layer. The polyurethane elastomers are typically produced by allowing a polyisocyanate, a polyol, and a chain extender to react with each other. The polyurethane elastomers are preferably block copolymers having a soft segment formed by a reaction between a polyol and a polyisocyanate and a hard segment formed by a reaction between a chain extender and a polyisocyanate.

Examples of the polyisocyanate include diphenylmethane diisocyanate, hexamethylene diisocyanate, tolidine diisocyanate, 1,5-naphthalene diisocyanate, isophorone diisocyanate, and xylylene diisocyanate. Among these, at least one of diphenylmethane diisocyanate or hexamethylene diisocyanate is preferred from the viewpoint of abrasion resistance, and isophorone diisocyanate is preferred from the viewpoint of disinfectant resistance.

Examples of the polyol include polytetramethylene ether glycol, polyester polyols, and lactone-based polyester polyols. Polyester polyols are produced by a polycondensation reaction between a dicarboxylic acid and a diol. Specific examples of the diol used in the production of polyester polyols include ethanediol, 1,3-propanediol, 1,4-butanediol, 1,5-pentanediol, and 1,6-hexanediol. These diols are used alone or in combination. Examples of the dicarboxylic acid include adipic acid and sebacic acid. These dicarboxylic acid are used alone or in combination.

Among these polyols, polytetramethylene ether glycol is preferred from the viewpoint of achieving high impact resilience.

Examples of the chain extender include aliphatic linear diols having 2 to 6 carbon atoms such as ethanediol, 1,4-butanediol, and 1,6-hexanediol; and 1,4-bis(hydroxyethoxy)benzene. Amines such as hexamethylenediamine, isophoronediamine, tolylenediamine, and monoethanolamine can be used in combination as needed. Among these, aliphatic linear diols having 2 to 6 carbon atoms are preferred from the viewpoint of abrasion resistance.

For the polyurethane elastomers according to the embodiment, disclosure of, for example, JP2005-015643A can be referred to. The polyurethane elastomer used in the present invention preferably has a polyester structure from the viewpoint of further enhancing the resilience.

The above polyurethane elastomers may be used alone or in combination of two or more thereof.

The content of the polyurethane elastomer in the resin cover layer in the case of a single-layer resin cover layer and the content of the polyurethane elastomer in the innermost layer in the case of a multilayered resin cover layer are each preferably 50% by mass or more, more preferably 70% by mass or more, still more preferably 80% by mass or more, and still more preferably 90% by mass or more. Alternatively, when the resin cover layer is formed of a single layer, the resin cover layer may be a layer composed of a polyurethane elastomer. When the resin cover layer is formed of multiple layers, the innermost layer may be a layer composed of a polyurethane elastomer.

When the resin cover layer in the case of a single-layer resin cover layer and the innermost layer in the case of a multilayered resin cover layer include a resin other than a polyurethane elastomer, the resin is not particularly limited as long as the effects of the present invention are not impaired. Examples of the resin include polyester elastomers and polyamide elastomers.

In the present invention, typical polyester elastomers that can be used for forming flexible tubes can be used as the polyester elastomers.

That is, the polyester elastomers used in the present invention are copolymers having a hard segment composed of a crystalline polyester and a soft segment composed of a polyether or a polyester.

Examples of the hard segment include polybutylene terephthalate and polyethylene terephthalate.

Examples of the soft segment include polyalkylene glycols such as polytetramethylene glycol and polypropylene glycol; bisphenol A-ethylene oxide adducts; bisphenol A-propylene oxide adducts; and polyesters such as polycaprolactone.

In the present invention, preferably, the "polyester elastomers" include neither a urethane bond nor an amide bond in the molecules thereof. The polyester elastomers may be used alone or in combination of two or more thereof.

Typical polyamide elastomers that can be used for forming flexible tubes can be used as the polyamide elastomers. In the present invention, preferably, the polyamide elastomers have no urethane bond. The polyamide elastomers may be used alone or in combination of two or more thereof.

When the resin cover layer is formed of multiple layers, a layer other than the innermost layer preferably includes at least one of a polyurethane elastomer, a polyamide elastomer, or a polyester elastomer. A layer having desired physical properties can be formed by appropriately combining these resins. When the resin cover layer is formed of multiple layers, the layer other than the innermost layer more preferably includes a polyurethane elastomer or an alloy of a polyurethane elastomer and a polyester elastomer from the viewpoint of further enhancing resilience.

A molecular weight of each of the elastomers that can be used in the resin cover layer of the present invention is preferably 10,000 to 1,000,000, more preferably 20,000 to 500,000, and particularly preferably 30,000 to 300,000.

In the present invention, the molecular weight of the elastomer refers to a weight-average molecular weight unless otherwise noted. The weight-average molecular weight can be measured by GPC as a molecular weight in terms of polystyrene. In this case, a GPC apparatus HLC-8220 (trade name, manufactured by Tosoh Corporation) is used; an eluant used is chloroform in the case of a polyester elastomer, NMP (N-methyl-2-pyrrolidone) in the case of a polyurethane elastomer, and m-cresol/chloroform (manufactured by Shonan Wako Junyaku K.K.) in the case of a polyamide elastomer; G3000HXL + G2000HXL (each of which is a trade name, manufactured by Tosoh Corporation) are used as columns; the temperature is 23°C; the flow rate is 1 mL/min; and the detection is performed with an RI detector.

As illustrated in Fig. 2, the resin cover layer 15 in the present invention is preferably formed so as to have a substantially uniform thickness in the longitudinal direction (axial direction) of the flexible tube base 14. The resin cover layer 15 has a thickness of, for example, 0.2 mm to 1.0 mm. The flexible tube 3a has an outer diameter D of, for example, 11 to 14 mm. In Fig. 2, the inner layer 17 and the outer layer 18 are formed such that a ratio of a thicknesses of the inner layer 17 to a total thickness of the resin cover layer 15 and a ratio of a thickness of the outer layer 18 to the total thickness of the resin cover layer 15 change in the axial direction of the flexible tube base 14. Specifically, on one end 14a side (distal end side) of the flexible tube base 14 attached to the angle portion 3b, the thickness of the inner layer 17 is larger than the thickness of the outer layer 18 with respect to the total thickness of the resin cover layer 15. The thickness of the inner layer 17 gradually decreases from the one end 14a toward the other end 14b side (proximal end side) attached to the main body operating section 5. On the other end 14b side, the thickness of the outer layer 18 is larger than the thickness of the inner layer 17.

In Fig. 2, the ratio of the thickness of the inner layer 17 is maximum on the one end 14a, and the ratio of the thickness of the outer layer 18 is maximum on the other end 14b. A ratio of the thickness of the inner layer 17 to the thickness of the outer layer 18 (thickness of inner layer 17:thickness of outer layer 18) can be, for example, 9:1 on the one end 14a, and, for example, 1:9 on the other end 14b. The thicknesses of the two layers are changed such that the ratio of the thickness of the inner layer 17 to the thickness of the outer layer 18 is reversed from the one end 14a to the other end 14b. With this configuration, the flexible tube 3a has a difference in hardness between the one end 14a side and the other end 14b side, and flexibility can be changed in the axial direction such that the one end 14a side is soft and the other end 14b side is hard. The inner layer and the outer layer are preferably formed such that the thickness ratio on the one end is 5:95 to 40:60 (inner layer:outer layer) and the thickness ratio on the other end is 95:5 to 60:40

(inner layer:outer layer).

When the ratio of the thickness of the inner layer 17 to the thickness of the outer layer 18 is within the range of 5:95 to 95:5, the amount of extrusion of a resin that forms a layer having a smaller thickness can also be accurately controlled.

The soft resin used in the inner layer 17 and the hard resin used in the outer layer 18 preferably have a difference in 100% modulus, which is an indicator indicating a hardness after molding, of 1 MPa or more and more preferably 3 MPa or more. A difference in melt viscosity, which is an indicator indicating a fluidity of a resin in a molten state, at a molding temperature of 150°C to 300°C is preferably 2,500 Pa·s or less. With this configuration, the resin cover layer 15 including the inner layer 17 and the outer layer 18 reliably achieves good molding accuracy and a necessary difference in hardness between the distal end side and the proximal end side.

## Topcoat Layer

In the flexible tube according to the present invention, the topcoat layer 16 is disposed on an outer periphery of the resin cover layer 15 as needed. Examples of the material of the topcoat layer include, but are not particularly limited to, urethane coatings, acrylic coatings, fluorine coatings, silicone coatings, epoxy coatings, and polyester coatings.

Main purposes of use of the topcoat layer are to protect the surface of the flexible tube, to make the surface of the flexible tube glossy, to impart slidability, and to impart chemical resistance. Therefore, the topcoat layer is preferably formed of a material that has a high modulus of elasticity, that provides a smooth surface, and that has good chemical resistance.

## Method for Producing Flexible Tube

### Formation of Primer Layer

In the production of the flexible tube base according to the present invention, first, a primer layer is formed on the flexible tube base. The primer layer can be formed by dissolving a compound represented by general formula (1) above in a solvent to prepare a coating liquid; forming a coating film on at least an outer periphery of the flexible tube base by, for example, applying or spraying the coating liquid onto the outer periphery of the flexible tube base or immersing the flexible tube base in the coating liquid; and subsequently drying the coating film by an ordinary method (for example, high-temperature drying at about 100°C).

Examples of the solvent used in the coating liquid include alcohol solvents such as methanol and ethanol; ketone solvents such as acetone and methyl ethyl ketone; ester solvents such as ethyl acetate; hydrocarbon solvents such as toluene; and liquid mixtures thereof. It is preferable to further mix water to the solvents in order to accelerate hydrolysis of the silane coupling agent. The coating liquid may be prepared to be acidic (for example, pH 1 to 4 at 25°C) or basic (for example, pH 9 to 11 at 25°C).

The content of the compound represented by general formula (1) in the coating liquid is not particularly limited, can be, for example, 0.01% by mass to 2% by mass, and is preferably 0.02% by mass or more and less than 0.5% by mass and more preferably 0.03% by mass or more and less than 0.4% by mass.

The coating liquid may include, for example, a surfactant and a catalyst besides the compound represented by general formula (1), the solvent, and a pH adjuster. The coating liquid is preferably constituted by the compound represented by general formula (1) and the solvent.

In the present invention, a portion that is not covered with the primer layer may be present on the outer periphery of the flexible tube base within a range that does not impair the effects of the present invention (that is, a defect may be partially generated in the primer layer).

Prior to the formation of the primer layer, the flexible tube base is preferably degreased with an alkali solution, an aqueous solution of a surfactant, an organic solvent, or the like. After the degreasing, the flexible tube base is preferably further washed with water or hot water.

### Formation of Resin Cover Layer

Formation of a resin cover layer will be described using, as an example, a case where the resin cover layer has a two-layer structure.

A flexible tube that includes a resin cover layer that has a two-layer structure having an inner layer and an outer layer can be produced by, for example, melt-kneading and extruding, around the flexible tube base on which the primer layer has been formed, a first resin material (resin material including a polyurethane elastomer) that forms the inner layer and a second resin material that forms the outer layer, thereby covering the flexible tube base.

In an embodiment in which a resin cover layer includes one layer or three or more layers, the resin cover layer

can also be produced by appropriately changing the layer configuration with reference to the method described below.

An example of a method for forming a resin cover layer of the flexible tube 3a (Figs. 1 and 2) will be described with reference to Figs. 3 and 4. In this embodiment, a continuous molding machine is used for molding a resin cover layer 15. It is preferable to use a continuous molding machine 20 that includes well-known extrusion units 21 and 22 including, hoppers, screws 21a and 22a, etc.; a head unit 23 configured to mold a resin cover layer 15 so as to cover an outer peripheral surface of a flexible tube base 14; a cooling unit 24; a transport unit 25 (including a supply drum 28 and a take-up drum 29) configured to transport a connected flexible tube base 31 to the head unit 23; and a control unit 26 configured to control the above units. The head unit 23 preferably includes a nipple 32, a die 33, and a support 34 configured to support the nipple 32 and the die 33 in a fixed manner. For example, the apparatus disclosed in Figs. 3 to 5 of JP2011-72391A can be used as an example of the apparatus having the above configuration.

The inside of the die 33 is preferably heated to a predetermined molding temperature. The molding temperature is preferably set in a range of 150°C to 300°C. By controlling a temperature of a heating unit in the apparatus by heating, temperatures of a first resin material 39 and a second resin material 40 can be made high. In addition to this, with an increase in the numbers of revolutions of the screws 21a and 22a, the temperatures of the first resin material 39 and the second resin material 40 can be further increased to enhance the fluidity of the respective resin materials. At this time, while a transport speed of the connected flexible tube base 31 is made constant, the amounts of ejection of the first resin material 39 and the second resin material 40 in the molten state are changed, thereby adjusting the molding thicknesses of an inner layer 17 and an outer layer 18.

A process of molding the resin cover layer 15 on the connected flexible tube base 31 by the continuous molding machine 20 will be described. When the continuous molding machine 20 performs a molding process, the first resin material 39 and the second resin material 40 in the molten state are respectively extruded from the extrusion units 21 and 22 to the head unit 23. In addition, the transport unit 25 operates so that the connected flexible tube base 31 is transported to the head unit 23. At this time, the extrusion units 21 and 22 are in a state of constantly extruding the first resin material 39 and the second resin material 40 to supply the resin materials 39 and 40 to the head unit 23, and the first resin material 39 and the second resin material 40 that are respectively extruded from the extrusion units 21 and 22 to gates 35 and 36 pass through edges and join to each other, and are supplied, in a state of being overlapped, through a resin passage 38 to a molding passage 37. As a result, a two-layer molded resin cover layer 15 is formed in which an inner layer 17 using the first resin material 39 and an outer layer 18 using the second resin material 40 overlap with each other.

The connected flexible tube base 31 includes a plurality of flexible tube bases 14 (each having a primer layer on the outer periphery thereof) that are connected together. While the connected flexible tube base 31 is transferred in the molding passage 37, a resin cover layer 15 is continuously molded on the plurality of flexible tube bases 14. When the resin cover layer 15 is molded from one end 14a side (distal end side) of one flexible tube base to the other end 14b side (proximal end side) thereof, immediately after start of the ejection of the resins by the extrusion units 21 and 22, the thickness of the inner layer 17 is made large. The ratio of the outer layer 18 is then gradually increased in an intermediate portion toward the other end 14b side. In this manner, the amounts of ejection of the resins are preferably controlled such that the resin cover layer 15 has the thickness ratio that changes in a gradient manner.

Joint members 30 each function as a connecting portion of two flexible tube bases 14, and thus the control unit 26 is used for switching the amounts of ejection of the extrusion units 21 and 22. Specifically, the control unit 26 preferably switches the amounts of ejection of the extrusion units 21 and 22 such that the thickness ratio changes from a thickness ratio on the other end 14b side (proximal end side) of one flexible tube base 14 to a thickness ratio on one end 14a side (distal end side) of a next flexible tube base 14. When the resin cover layer 15 is molded from the one end 14a side of the next flexible tube base 14 to the other end 14b side thereof, the extrusion units 21 and 22 are preferably similarly controlled such that the thickness of the outer layer gradually increases from the one end side toward the other end side.

The connected flexible tube base 31 on which the resin cover layer 15 is molded to the backmost end is removed from the continuous molding machine 20, and the joint members 30 are then removed from the flexible tube bases 14 to separate the flexible tube bases 14 from each other. Next, for each of the separated flexible tube bases 14, the resin cover layer 15 is coated with a topcoat layer 16 to complete flexible tubes 3a. The completed flexible tubes 3a are transferred to an assembly process of an electronic endoscope.

In the present invention, when the resin cover layer is formed of multiple layers, a functional layer may be disposed between layers that form the multiple layers.

The above description has been made with reference to the drawings using, as an example, an electronic endoscope configured to observe an image of a state of a subject taken by using an imaging device. However, the present invention is not limited thereto and is also applicable to an endoscope configured to observe a state of a subject by employing an optical image guide.

The flexible tube according to the present invention is widely applicable to endoscopic medical devices. For example, the flexible tube is applicable to an instrument that includes an endoscope having a clip or wire at a tip thereof or an instrument that includes an endoscope having a basket or brush at a tip thereof. Note that the endoscopic medical

device broadly encompasses, besides the above-described medical device that includes an endoscope as a basic structure, a medical device or diagnosis and treatment device that includes an insertion section having flexibility and is used by being introduced into the body, such as a remote control medical device.

In the endoscopic medical device according to the present invention, the flexible tube for an endoscope according to the present invention is incorporated in an insertion section of the endoscopic medical device. That is, a method for producing an endoscopic medical device according to the present invention includes incorporating the flexible tube for an endoscope according to the present invention into an insertion section of an endoscopic medical device.

## EXAMPLES

Hereafter, the present invention will be described in more detail by way of Examples. However, it is to be understood that the present invention is not limited by these Examples. Preparation of Coating Liquid for Forming Primer Layer

A solution having a ratio water/ethanol of 5/75 on a mass basis was prepared. The compound described in the table below was dissolved in the solution so as to have a concentration of 8.9 g/kg, and the resulting solution was used as a coating liquid for forming a primer layer.

Preparation of Coating Liquid for Forming Adhesive Layer

In 1 kg of methyl ethyl ketone, 100 g of a polyester polyurethane (trade name: N-2304, manufactured by Nippon Polyurethane Industry Co., Ltd.) and 10 g of a polyisocyanate (trade name: CORONATE, manufactured by Nippon Polyurethane Industry Co., Ltd) were dissolved to prepare a coating liquid for forming an adhesive layer.

Production of Flexible Tube for Endoscope

A flexible tube having the structure illustrated in Fig. 2 was prepared. The resin cover layer had a single-layer structure or a two-layer structure as shown in the table below. Flexible Tube Base

A spiral tube 11 was formed by using a metal strip 11a made of stainless steel, and a flexible tube base having a form in which the spiral tube 11 was covered with a cylindrical mesh 12 obtained by weaving stainless steel fibers was prepared. This flexible tube base has a length of 80 cm and a diameter of 12 mm. This stainless steel flexible tube has a passivation layer on a surface thereof, the passivation layer being formed by an annealing treatment (heating treatment) in the formation of the spiral tube and the cylindrical mesh.

Formation of Primer Layer

The flexible tube base was washed by immersing in a 7.5% aqueous solution of sodium hydroxide at 60°C for one minute. Subsequently, the flexible tube base was rinsed with distilled water and then dried in an oven at 100°C for 10 minutes. The washed flexible tube base was immersed in the above-prepared coating liquid for forming a primer layer at room temperature for one minute and then dried in an oven at 160°C for 10 minutes. Thus, a flexible tube base having a primer layer on the outer periphery thereof (the surface to be covered with a resin) was prepared.

Formation of Adhesive Layer

The above-prepared coating liquid for forming an adhesive layer was uniformly applied to the outer periphery of the stainless steel flexible tube base and dried at room temperature for two hours. Subsequently, heat treatment was further performed at 150°C for two hours to prepare a flexible tube base having an adhesive layer on the outer periphery thereof (the surface to be covered with a resin) thereof. The adhesive layer had a thickness of about 80 $\mu$m.

Formation of Resin Cover Layer

The outer periphery of the flexible tube base having the primer layer or the adhesive layer was covered with the resin described in Table 1 below by extrusion (molding temperature: 200°C) to prepare a flexible tube for an endoscope, the flexible tube having a resin cover layer. The resin cover layer had a thickness of 0.4 mm (in the case of a two-layer structure, the total thickness of the two layers was 0.4 mm).

In the cases where the resin cover layer is formed of two layers (Examples 9 to 12 and Comparative Examples 2 and 7), the two layers were simultaneously molded by two-layer extrusion molding to cover the flexible tube base. In these cases, the inner/outer layer ratio on the distal end and the inner/outer layer ratio on the proximal end were inner layer:outer layer = 80:20 on the distal end and inner layer:outer layer = 20:80 on the proximal end, respectively.

[Test Example 1] Evaluation of Adhesiveness between Flexible Tube Base and Resin Cover Layer

A slit having a width of 1 cm was cut in the resin cover layer of the above-prepared flexible tube for an endoscope in the axial direction of the flexible tube. A 90° peel strength was measured by peeling from the slit having a width of 1 cm between the flexible tube base and the resin cover layer (the innermost layer in the case of two layers). The peel strength was measured with a force gauge. The measured 90° peel strength was evaluated on the basis of the evaluation criteria described below.

Evaluation Criteria for Adhesiveness

A: The 90° peel strength is 15 N/cm or more.
B: The 90° peel strength is 10 N/cm or more and less than 15 N/cm.
C: The 90° peel strength is 5 N/cm or more and less than 10 N/cm.
D: The 90° peel strength is less than 5 N/cm.
The results are shown in the table below.

[Test Example 2] Evaluation of Resilience

In an environment at a temperature of 25°C and a relative humidity of 50%, positions of 30 cm and 50 cm from one tip portion of the above-prepared flexible tube for an endoscope were fixed. A position of 40 cm (central portion of the flexible tube) was pushed by 15 mm in a direction (diameter direction) perpendicular to the length direction of the flexible tube. A ratio of a repulsive force (b) after 30 seconds to a repulsive force (a) after 0.1 seconds was measured as a resilience (%). The repulsive force was measured with a force gauge (ZTS50N, manufactured by IMADA CO., LTD.).

$$[\text{Resilience } (\%)] = [(b)/(a)] \times 100$$

The resilience was evaluated on the basis of the evaluation criteria described below.

Evaluation Criteria for Resilience

A: The resilience is 80% or more.
B: The resilience is 75% or more and less than 80%.
C: The resilience is 65% or more and less than 75%.
D: The resilience is less than 65%.

The results are shown in the table below.

Table 1

| | | Example | | | | | | | | | | | | Comparative Example | | | | | | | | |
|---|---|---|---|---|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 1 | 2 | 3 | 4 | 5 | 6 | 7 | 8 | 9 |
| Resin cover layer | Inner layer | PU1 | PU2 | PU3 | PU4 | PU1 | PU2 | PU3 | PU4 | PU1 | PU2 | PU3 | PU4 | PU1 | PU3 | PU1 | PU1 | PU2 | PU3 | PU1 | Fluororesin | Olefin resin |
| | Outer layer | | | | | | | | | PU5 | PU5 | PU5 | PU5 | | PU5 | | | | | PU6 | | |
| Compound of primer layer | | S-1 | S-2 | S-6 | S-7 | S-12 | S-33 | S-34 | S-37 | S-1 | S-3 | S-13 | S-32 | Adhesive layer | Adhesive layer | B-1 | B-2 | B-3 | B-4 | B-5 | S-1 | S-2 |
| Evaluation | Adhesiveness | A | A | B | B | A | A | B | B | A | A | B | B | D | D | C | D | C | C | C | C | C |
| | Resilience | B | B | B | A | B | B | B | A | A | A | A | A | C | C | C | D | D | C | D | D | D |

PU1 Polyurethane elastomer (trade name: Miractran E375, manufactured by Nippon Miractran Co., Ltd.)
PU2 Polyurethane elastomer (trade name: Miractran E675, manufactured by Nippon Miractran Co., Ltd.)
PU3 Polyurethane elastomer (trade name: PANDEX T5875, manufactured by DIC Corporation)
PU4 Polyurethane elastomer (trade name: PANDEX T8185, manufactured by DIC Corporation)
PU5 Polyurethane elastomer (trade name: PANDEX T2190, manufactured by DIC Corporation)
B-1 3-Glycidoxypropyltrimethoxysilane
B-2 Tetra-n-butoxytitanium
B-3 3-Methacryloyloxypropyltrimethoxysilane
B-4 3-Mercaptopropyltrimethoxysilane
B-5 Tetraethyl orthosilicate (TEOS, tetraethoxysilane)
Fluororesin Fluorine elastomer (trade name: DAI-EL T-530, manufactured by Daikin Industries, Ltd.)
Olefin resin Olefin elastomer (trade name: ZELAS MC707, manufactured by Mitsubishi Chemical Corporation)
Adhesive layer Polyester polyurethane (trade name: N-2304, manufactured by Nippon Polyurethane Industry Co., Ltd.):Polyisocyanate (trade name: CORONATE, manufactured by Nippon Polyurethane Industry Co., Ltd.) = 10:1 (mass ratio)

In Table 1 above, S-1, S-2, S-3, S-6, S-7, S-12, S-13, S-32, S-33, S-34, and S-37 are respectively compounds S-1, S-2, S-3, S-6, S-7, S-12, S-13, S-32, S-33, S-34, and S-37 shown as the specific examples described above.

As shown in Table 1, in the cases where an existing adhesive layer was formed instead of the primer layer on the outer periphery of the flexible tube base, and the periphery of the adhesive layer was covered with a polyurethane elastomer, the results of both the adhesiveness and resilience were poor (Comparative Examples 1 and 2).

Similarly, in the cases where compounds that were not included in compounds represented by general formula (1) (coupling agents having functional groups other than an amino group) were used as the primer layer, the results of both the characteristics of adhesiveness and resilience were poor (Comparative Examples 3 to 7). Tetraethyl orthosilicate, which was used in the formation of the primer layer in Comparative Example 7, forms the primer layer in a state where ethoxy groups bonded to the silicon atom are hydrolyzed and the silicon atom has hydroxy groups.

Furthermore, even in the cases where compounds represented by general formula (1) were used as the primer layer, both the characteristics of adhesiveness and resilience were also poor when the resin cover layer in contact with the primer layer included no polyurethane elastomer (Comparative Examples 8 and 9).

In contrast, in the cases where compounds represented by general formula (1) were used as the primer layer, and a polyurethane elastomer was used as the resin cover layer in contact with the primer layer, the resulting flexible tubes each had good adhesiveness between the base and the resin cover layer and also had good resilience (Examples 1 to 12).

Reference Signs List

| | |
|---|---|
| 2 | electronic endoscope (endoscope) |
| 3 | insertion section |
| | 3a flexible tube |
| | 3b angle portion |
| | 3c tip portion |
| 5 | main body operating section |
| 6 | universal cord |
| 11 | spiral tube |
| | 11a metal strip |
| 12 | cylindrical mesh |
| 13 | cap |
| 14 | flexible tube base |
| | 14a distal end side |
| | 14b proximal end side |
| 15 | resin cover layer |
| 16 | topcoat layer |
| 17 | inner layer |
| 18 | outer layer |
| X | angle portion 3b side (soft) |
| Y | main body operating section 5 side (hard) |
| 20 | continuous molding machine (production apparatus) |
| 21, 22 | extrusion unit |
| | 21a screw |
| | 22a screw |
| 23 | head unit |
| 24 | cooling unit |
| 25 | transport unit |
| 26 | control unit |
| 28 | supply drum |
| 29 | take-up drum |
| 30 | joint member |
| 31 | connected flexible tube base |
| 32 | nipple |
| 33 | die |
| 34 | support |
| 35, 36 | gate |

37 molding passage
38 resin passage
39 soft resin (including polyurethane elastomer)
40 hard resin

## Claims

1. A flexible tube for an endoscope, the flexible tube comprising:

   a flexible tube base containing metal as a constituent material;
   a resin cover layer that covers an outer periphery of the flexible tube base; and
   a primer layer that includes a compound represented by general formula (1) below and that is disposed between the flexible tube base and the resin cover layer,
   wherein the resin cover layer includes a polyurethane elastomer at least on a side in contact with the primer layer:

   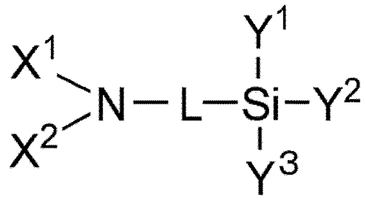

   General formula (1)

   where $X^1$ and $X^2$ each represent a hydrogen atom, an alkyl group, a cycloalkyl group, an alkenyl group, an aryl group, an acyl group, an alkoxycarbonyl group, or a carbamoyl group,
   $Y^1$ represents a hydroxy group or an alkoxy group, $Y^2$ and $Y^3$ each represent a hydroxy group, an alkoxy group, or an alkyl group, and
   L represents a single bond, a divalent group selected from the group consisting of an alkylene group, an arylene group, and -O-, or a divalent group which is a combination of two or more divalent groups selected from the group consisting of an alkylene group, an arylene group, and -O-.

2. The flexible tube for an endoscope according to claim 1, wherein the metal that constitutes the flexible tube base is stainless steel.

3. The flexible tube for an endoscope according to claim 1 or 2, wherein the metal that constitutes the flexible tube base has a passivation film on a surface of the flexible tube base.

4. The flexible tube for an endoscope according to any one of claims 1 to 3, wherein the resin cover layer has a single-layer structure or a multilayer structure and includes the polyurethane elastomer in a layer in contact with the primer layer.

5. The flexible tube for an endoscope according to any one of claims 1 to 4, wherein the resin cover layer has a two-layer structure, and a ratio of a thickness of an inner layer to a thickness of an outer layer of the two-layer structure changes in a gradient manner in an axial direction of the flexible tube base.

6. The flexible tube for an endoscope according to claim 5, wherein the ratio of the thickness of the inner layer to the thickness of the outer layer is inner layer:outer layer = 5:95 to 40:60 on one end of the flexible tube for an endoscope and inner layer:outer layer = 95:5 to 60:40 on the other end.

7. An endoscopic medical device comprising the flexible tube for an endoscope according to any one of claims 1 to 6.

8. A method for producing a flexible tube for an endoscope, the method comprising:

   a step of forming, on at least an outer periphery of a flexible tube base that contains metal as a constituent material, a primer layer that includes a compound represented by general formula (1) below; and
   a step of forming a resin cover layer by covering, with a resin that includes a polyurethane elastomer, the primer

layer formed on the outer periphery of the flexible tube base so as to be in contact with the primer layer:

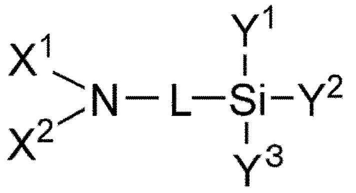

General formula (1)

where $X^1$ and $X^2$ each represent a hydrogen atom, an alkyl group, a cycloalkyl group, an alkenyl group, an aryl group, an acyl group, an alkoxycarbonyl group, or a carbamoyl group,
$Y^1$ represents a hydroxy group or an alkoxy group, $Y^2$ and $Y^3$ each represent a hydroxy group, an alkoxy group, or an alkyl group, and
L represents a single bond, a divalent group selected from the group consisting of an alkylene group, an arylene group, and -O-, or a divalent group which is a combination of two or more divalent groups selected from the group consisting of an alkylene group, an arylene group, and -O-.

**9.** The method for producing a flexible tube for an endoscope according to claim 8, wherein
the resin cover layer has a two-layer structure,
at least an inner layer of the two-layer structure includes a polyurethane elastomer, and a ratio of a thickness of the inner layer to a thickness of an outer layer of the two-layer structure changes in a gradient manner in an axial direction of the flexible tube base.

**10.** A method for producing an endoscopic medical device, comprising:

a step of producing a flexible tube for an endoscope by the method for producing a flexible tube for an endoscope according to claim 8 or 9; and
a step of incorporating the produced flexible tube for an endoscope into an insertion section of an endoscopic medical device.

**11.** A method for producing an endoscopic medical device, comprising incorporating the flexible tube for an endoscope according to any one of claims 1 to 6 into an insertion section of an endoscopic medical device.

**Patentansprüche**

**1.** Flexibler Schlauch für ein Endoskop, wobei der flexible Schlauch umfasst:

eine flexible Schlauchbasis, die Metall als ein Grundmaterial enthält;
eine Harzabdeckungsschicht, die einen Außenumfang der flexiblen Schlauchbasis abdeckt; und
eine Grundierungsschicht, die eine Verbindung beinhaltet, die durch die allgemeine Formel (1) unten dargestellt wird, und die zwischen der flexiblen Schlauchbasis und der Harzabdeckungsschicht angeordnet ist,
wobei die Harzabdeckungsschicht ein Polyurethanelastomer mindestens an einer Seite in Kontakt mit der Grundierungsschicht beinhaltet:

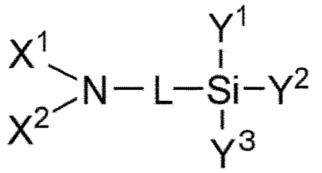

Allgemeine Formel (1)

wobei $X^1$ und $X^2$ jeweils ein Wasserstoffatom, eine Alkylgruppe, eine Cycloalkylgruppe, eine Alkenylgruppe, eine Arylgruppe, eine Acylgruppe, eine Alkoxycarbonylgruppe oder eine Carbamoylgruppe darstellen,
$Y^1$ eine Hydroxygruppe oder eine Alkoxygruppe darstellt, $Y^2$ und $Y^3$ jeweils eine Hydroxygruppe, eine Alkoxygruppe oder eine Alkylgruppe darstellen, und
L eine Einfachbindung, eine divalente Gruppe, ausgewählt aus der Gruppe bestehend aus einer Alkylengruppe, einer Arylengruppe und -O-, oder eine divalente Gruppe, die eine Kombination von zwei oder mehr divalenten Gruppen, ausgewählt aus der Gruppe bestehend aus einer Alkylengruppe, einer Arylengruppe und -O-, ist, darstellt.

2. Flexibler Schlauch für ein Endoskop nach Anspruch 1, wobei das Metall, das die flexible Schlauchbasis begründet, rostfreier Stahl ist.

3. Flexibler Schlauch für ein Endoskop nach Anspruch 1 oder 2, wobei das Metall, das die flexible Schlauchbasis begründet, einen Passivierungsfilm an einer Oberfläche der flexiblen Schlauchbasis aufweist.

4. Flexibler Schlauch für ein Endoskop nach einem der Ansprüche 1 bis 3, wobei die Harzabdeckungsschicht eine Einzelschichtstruktur oder eine Mehrfachschichtstruktur aufweist und das Polyurethanelastomer in einer Schicht in Kontakt mit der Grundierungsschicht beinhaltet.

5. Flexibler Schlauch für ein Endoskop nach einem der Ansprüche 1 bis 4, wobei die Harzabdeckungsschicht eine Zweischichtstruktur aufweist und ein Verhältnis einer Dicke einer Innenschicht zu einer Dicke einer Außenschicht der Zweischichtstruktur sich schrittweise in einer Achsrichtung der flexiblen Schlauchbasis ändert.

6. Flexibler Schlauch für ein Endoskop nach Anspruch 5, wobei das Verhältnis der Dicke der Innenschicht zu der Dicke der Außenschicht Innenschicht:Außenschicht = 5:95 bis 40:60 an einem Ende des flexiblen Schlauchs für ein Endoskop und Innenschicht:Außenschicht = 95:5 bis 60:40 an dem anderen Ende ist.

7. Endoskopische medizinische Vorrichtung, umfassend den flexiblen Schlauch für ein Endoskop nach einem der Ansprüche 1 bis 6.

8. Verfahren zum Herstellen eines flexiblen Schlauchs für ein Endoskop, wobei das Verfahren umfasst:

einen Schritt zum Bilden, an mindestens einem Außenumfang einer flexiblen Schlauchbasis, die Metall als ein Grundmaterial enthält, einer Grundierungsschicht, die eine Verbindung beinhaltet, die von der allgemeinen Formel (1) unten dargestellt wird; und
einen Schritt zum Bilden einer Harzabdeckungsschicht durch Abdecken, mit einem Harz, das ein Polyurethanelastomer beinhaltet, der Grundierungsschicht, die an dem Außenumfang der flexiblen Schlauchbasis gebildet ist, um in Kontakt mit der Grundierungsschicht zu sein:

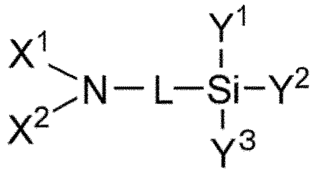

Allgemeine Formel (1)

wobei $X^1$ und $X^2$ jeweils ein Wasserstoffatom, eine Alkylgruppe, eine Cycloalkylgruppe, eine Alkenylgruppe, eine Arylgruppe, eine Acylgruppe, eine Alkoxycarbonylgruppe oder eine Carbamoylgruppe darstellen,
$Y^1$ eine Hydroxygruppe oder eine Alkoxygruppe darstellt, $Y^2$ und $Y^3$ jeweils eine Hydroxygruppe, eine Alkoxygruppe oder eine Alkylgruppe darstellen, und
L eine Einfachbindung, eine divalente Gruppe, ausgewählt aus der Gruppe bestehend aus einer Alkylengruppe, einer Arylengruppe und -O-, oder eine divalente Gruppe, die eine Kombination von zwei oder mehr divalenten Gruppen, ausgewählt aus der Gruppe bestehend aus einer Alkylengruppe, einer Arylengruppe und -O-, ist, darstellt.

9. Verfahren zum Herstellen eines flexiblen Schlauchs für ein Endoskop nach Anspruch 8, wobei
die Harzabdeckungsschicht eine Zweischichtstruktur aufweist,
mindestens eine Innenschicht der Zweischichtstruktur ein Polyurethanelastomer beinhaltet, und
ein Verhältnis einer Dicke der Innenschicht zu einer Dicke einer Außenschicht der Zweischichtstruktur sich schrittweise in einer Achsrichtung der flexiblen Schlauchbasis ändert.

**10.** Verfahren zum Herstellen einer endoskopischen medizinischen Vorrichtung, umfassend:

einen Schritt zum Herstellen eines flexiblen Schlauchs für ein Endoskop durch das Verfahren zum Herstellen eines flexiblen Schlauchs für ein Endoskop nach Anspruch 8 oder 9; und
einen Schritt zum Eingliedern des hergestellten flexiblen Schlauchs für ein Endoskop in einen Einsatzabschnitt einer endoskopischen medizinischen Vorrichtung.

**11.** Verfahren zum Herstellen einer endoskopischen medizinischen Vorrichtung, umfassend Eingliedern des flexiblen Schlauchs für ein Endoskop nach einem der Ansprüche 1 bis 6 in einen Einsatzabschnitt einer endoskopischen medizinischen Vorrichtung.

**Revendications**

**1.** Tube flexible pour un endoscope, le tube flexible comprenant :

une base de tube flexible contenant du métal en tant que matériau constituant ;
une couche de couverture en résine qui recouvre une périphérie extérieure de la base de tube flexible ; et
une couche d'apprêt qui inclut un composé représenté par la formule générale (1) ci-dessous et qui est disposée entre la base de tube flexible et la couche de couverture en résine,
dans lequel la couche de couverture en résine inclut un élastomère de polyuréthane sur au moins un côté en contact avec la couche d'apprêt :

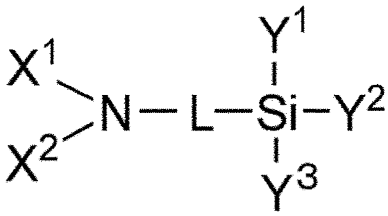
Formule générale (1)

où $X^1$ et $X^2$ représentent chacun un atome d'hydrogène, un groupe alkyle, un groupe cycloalkyle, un groupe alcényle, un groupe aryle, un groupe acyle, un groupe alcoxycarbonyle, ou un groupe carbamoyle,
$Y^1$ représente un groupe hydroxy ou un groupe alcoxy, $Y^2$ et $Y^3$ représentent chacun un groupe hydroxy, un groupe alcoxy, ou un groupe alkyle, et
L représente une liaison unique, un groupe divalent choisi parmi le groupe consistant en un groupe alkylène, un groupe arylène, et -O-, ou un groupe divalent qui est une combinaison de deux ou plus de deux groupes divalents choisis parmi le groupe consistant en un groupe alkylène, un groupe arylène, et -O-.

**2.** Tube flexible pour un endoscope selon la revendication 1, dans lequel le métal qui constitue la base de tube flexible est de l'acier inoxydable.

**3.** Tube flexible pour un endoscope selon la revendication 1 ou 2, dans lequel le métal qui constitue la base de tube flexible présente un film de passivation sur une surface de la base de tube flexible.

**4.** Tube flexible pour un endoscope selon l'une quelconque des revendications 1 à 3, dans lequel la couche de couverture en résine présente une structure monocouche ou une structure multicouche et inclut l'élastomère de polyuréthane dans une couche en contact avec la couche d'apprêt.

**5.** Tube flexible pour un endoscope selon l'une quelconque des revendications 1 à 4, dans lequel la couche de couverture en résine présente une structure à deux couches, et un rapport entre une épaisseur d'une couche intérieure et une épaisseur d'une couche extérieure de la structure à deux couches change d'une manière progressive dans une direction axiale de la base de tube flexible.

**6.** Tube flexible pour un endoscope selon la revendication 5, dans lequel le rapport entre l'épaisseur de la couche intérieure et l'épaisseur de la couche extérieure est couche intérieure :couche extérieure = 5:95 à 40:60 sur une extrémité du tube flexible pour un endoscope et couche intérieure :couche extérieure = 95:5 à 60:40 sur l'autre

extrémité.

7. Dispositif médical endoscopique comprenant le tube flexible pour un endoscope selon l'une quelconque des revendications 1 à 6.

8. Procédé de production d'un tube flexible pour endoscope, le procédé comprenant :

une étape de formation, sur au moins une périphérie extérieure d'une base de tube flexible qui contient du métal en tant que matériau constituant, d'une couche d'apprêt qui inclut un composé représenté par la formule générale (1) ci-dessous ; et
une étape de formation d'une couche de couverture en résine en recouvrant, avec une résine qui inclut un élastomère de polyuréthane, la couche d'apprêt formée sur la périphérie extérieure de la base de tube flexible de manière à être en contact avec la couche d'apprêt :

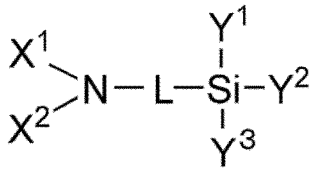
Formule générale (1)

où $X^1$ et $X^2$ représentent chacun un atome d'hydrogène, un groupe alkyle, un groupe cycloalkyle, un groupe alcényle, un groupe aryle, un groupe acyle, un groupe alcoxycarbonyle, ou un groupe carbamoyle,
$Y^1$ représente un groupe hydroxy ou un groupe alcoxy, $Y^2$ et $Y^3$ représentent chacun un groupe hydroxy, un groupe alcoxy, ou un groupe alkyle, et
L représente une liaison unique, un groupe divalent choisi parmi le groupe consistant en un groupe alkylène, un groupe arylène, et -O-, ou un groupe divalent qui est une combinaison de deux ou plus de deux groupes divalents choisis parmi le groupe consistant en un groupe alkylène, un groupe arylène, et -O-.

9. Procédé de production d'un tube flexible pour un endoscope selon la revendication 8, dans lequel
la couche de couverture en résine présente une structure à deux couches,
au moins une couche intérieure de la structure à deux couches inclut un élastomère de polyuréthane, et
un rapport entre une épaisseur de la couche intérieure et une épaisseur d'une couche extérieure de la structure à deux couches change d'une manière progressive dans une direction axiale de la base de tube flexible.

10. Procédé de production d'un dispositif médical endoscopique, comprenant :

une étape de production d'un tube flexible pour un endoscope par le procédé de production d'un tube flexible pour un endoscope selon la revendication 8 ou 9 ; et
une étape d'incorporation du tube flexible produit pour un endoscope dans une section d'insertion d'un dispositif médical endoscopique.

11. Procédé de production d'un dispositif médical endoscopique, comprenant l'incorporation du tube flexible pour un endoscope selon l'une quelconque des revendications 1 à 6 dans une section d'insertion d'un dispositif médical endoscopique.

FIG. 1

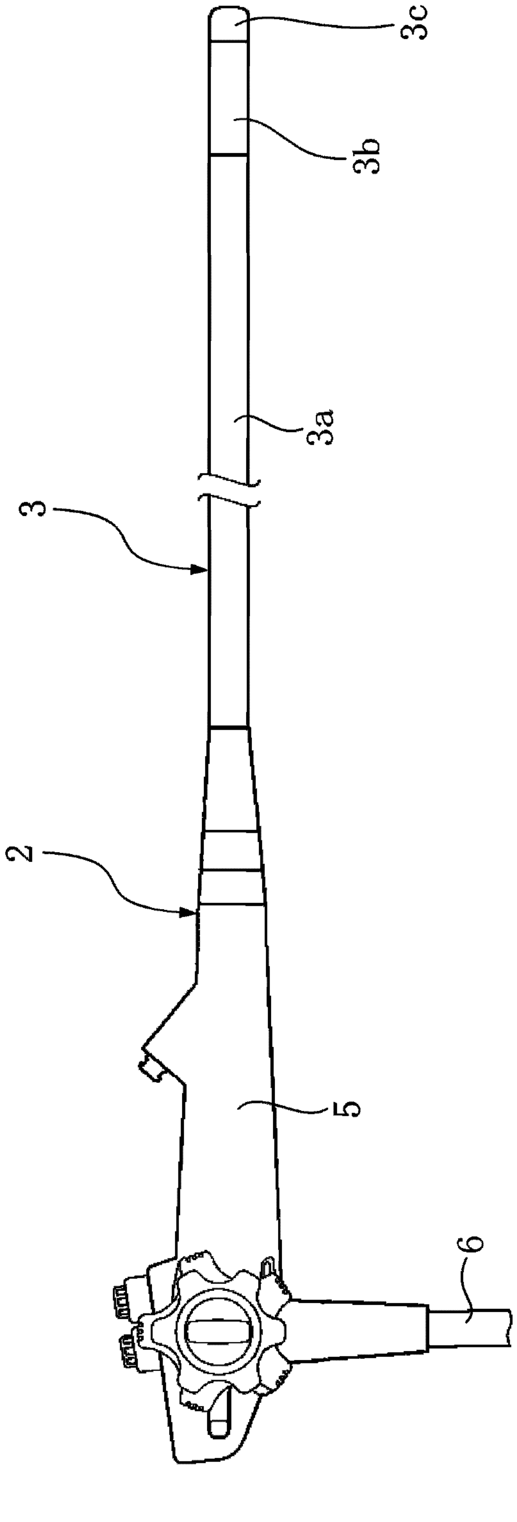

FIG. 2

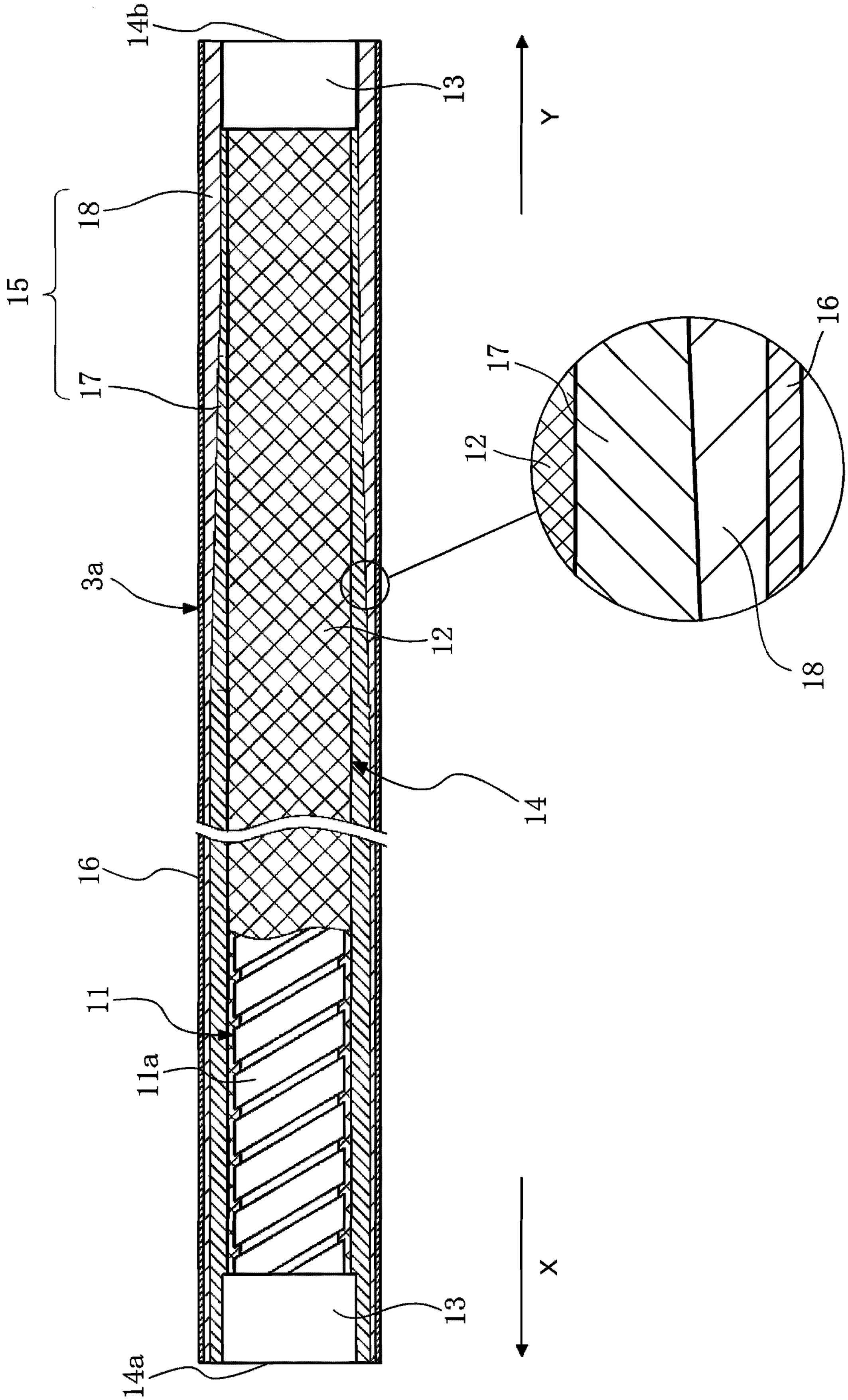

FIG. 3

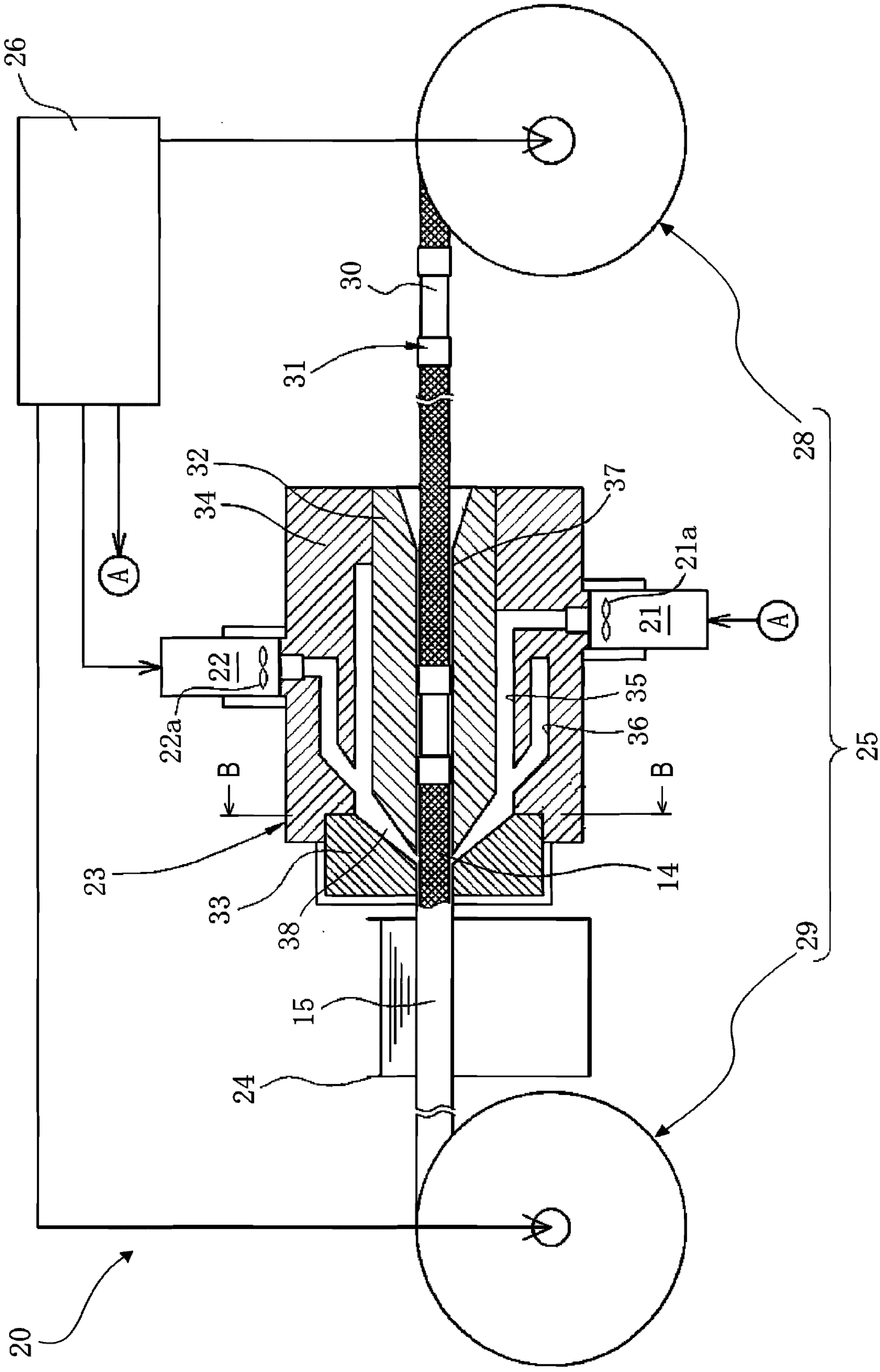
26
30
31
28
32
34
37
21a
21
A
A
22
22a
35
36
B
B
25
23
14
33
38
29
15
24
20

FIG. 4

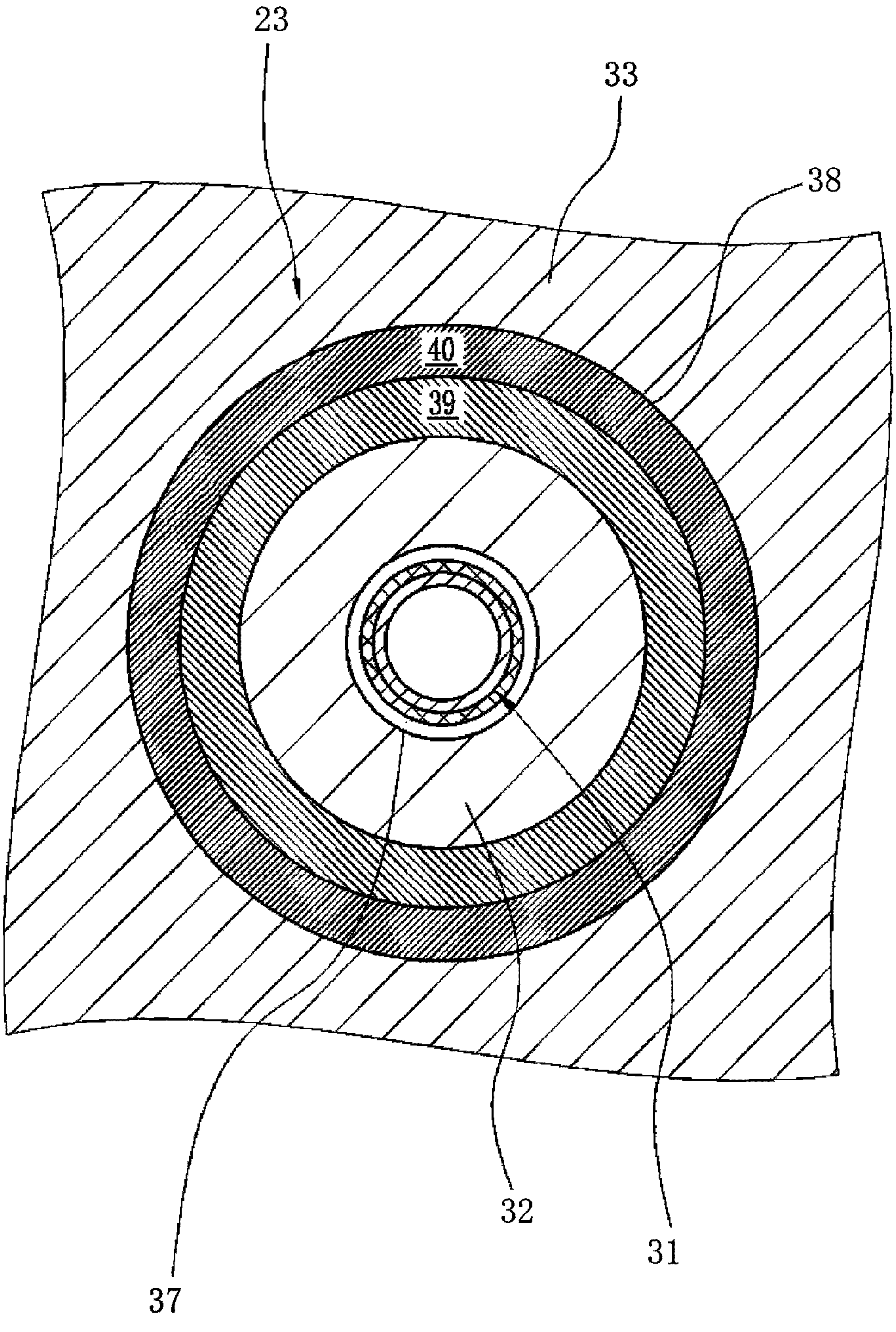

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11042205 A
- JP H1142205 A
- EP 3000834 A1
- US 6350799 B1
- JP 2004141487 A
- JP 2005015643 A
- JP 2011072391 A